# EUROPEAN PATENT SPECIFICATION

(11) **EP 1 576 134 B1**
(45) Date of publication and mention of the grant of the patent: **06.03.2013**
(21) Application number: 03790356.4
(22) Date of filing: 05.12.2003
(51) Int. Cl.: G01N 33/50, G01N 33/68

(54) **METHOD FOR DISCOVERING NEUROGENIC AGENTS**
VERFAHREN ZUR ENTDECKUNG NEUROGENER WIRKSTOFFE
PROCEDE DE DECOUVERTE D'AGENTS NEUROGENES

(30) Priority: 09.12.2002 US 432359 P; 08.08.2003 US 493674 P
(43) Date of publication of application: 21.09.2005
(73) Proprietor: Kelleher-Andersson, Judith, Columbia, MD 21044 (US); Johe, Karl K., Potomac, MD 20854 (US)
(72) Inventor: Kelleher-Andersson, Judith, Columbia, MD 21044 (US); Johe, Karl K., Potomac, MD 20854 (US)
(74) Representative: Becker Kurig Straus
(86) International application number: PCT/US2003/038670
(87) International publication number: WO 2004/053071

(56) References cited:
- WO-A2-00/09676
- WO-A2-00/52143
- WO-A2-01/88104
- WO-A2-02/058736
- US-A- 5 750 376
- US-A- 6 040 180
- US-B1- 6 531 464
- DOWLING-WARRINER C V ET AL: "Induction of gap junctional intercellular communication, connexin43 expression, and subsequent differentiation in human fetal neuronal cells by stimulation of the cyclic AMP pathway" NEUROSCIENCE, vol. 95, no. 3, 8 December 2000 (2000-12-08), pages 859-868, XP009078099 ISSN: 0306-4522
- MALBERG JESSICA E ET AL: "Chronic antidepressant treatment increases neurogenesis in adult rat hippocampus" JOURNAL OF NEUROSCIENCE, vol. 20, no. 24, 15 December 2000 (2000-12-15), pages 9104-9110, XP009077904 ISSN: 0270-6474

## Description

### BACKGROUND OF THE INVENTION

### 1. FIELD OF THE INVENTION

According to a long-held doctrine, no significant number of neurons are made and contribute to function in the adult mammalian nervous system. However, recent data indicate that adult mammalian brains contain neural precursor cells capable of generating new neurons both in normal and in injured conditions. These new neurons have been quantified in live animals by injecting or feeding in drinking water a marker of dividing cells, bromodeoxyuridine (BrdU) and by immunostaining of post-mortem brains with antibodies against BrdU and neuronal markers. An endogenous marker of dividing cells, ki67 protein, has also been used instead of BrdU for this purpose. Thus, in healthy, young rodents, approximately 3,000 - 15,000 new cells per day are estimated to be born in the dentate gyrus of the hippocampus, about 60% of which express early neuron-specific proteins such as double-cortin and type III beta-tubulin. Significant number of new cells and new neurons have also been observed in healthy, young primates. In rodents as well as in primates, the location of neurogenic areas in the CNS is limited to the dentate gyrus of the hippocampus and the subependymal layer of the striatum. In human patients of different ages who have been diagnosed with a tumor of the tongue, a single injection of BrdU has revealed significant number of new cells and new neurons being born in the dentate gyrus and the subependymal layer of the striatum. Thus, the process of generating new neurons (neurogenesis) occurs in the mature, adult brain in significant quantities throughout rodents, primates, and human species.

Such significant quantities of new neurons suggest that they may be important for the normal physiology of the brain, especially the hippocampus. Hippocampus is the well-known center of learning, memory, and other cognitive functions, processes which new information are added, edited, stored, and recalled constantly throughout life. Since hippocampus is also the most potent neurogenic area of the brain, many studies have been undertaken to establish whether neurogenesis may be the cellular mechanism to structurally accommodate the ever-increasing volume of cognitive processing to be handled. Thus, it has been shown that at least some of the newly born neurons, marked by genetic markers, do mature to be electrophysiologically active and integrate into the existing neuronal circuitry of the hippocampus. Ablation of the neurogenesis in rats leads to decreased cognitive capabilities in several behavior tests. Thus, the existing data demonstrate that neurogenesis significantly contributes to the normal hippocampal physiology.

In abnormal conditions, such as when an injury to a brain area has occurred, neurogenesis becomes more widespread and perhaps functionally diverse. In rodent models of ischemic and hemorrhagic stroke, the newly born neurons of the subependyma (also referred to as subventricular zone) are seen migrating to and accumulating in the lesion area of the cortex. However, the newly born neurons have short survival period.

Thus, a compound that can stimulate the endogenous neurogenesis either in a disease state or in a healthy state may be an effective drug for a number of human nervous system diseases. However, the current limitation is the lack of an effective, predictive in vitro assay that can be used to select a neurogenic compound for clinical drug development. Disclosed here is a novel, in vitro assay, which is effective and predictive, to be useful for discovering a compound that promotes neurogenesis in vivo. Also disclosed are classes of compound structures that are shown to be particularly effective in promoting the neurogenesis.

This invention relates to a method and an assay for identifying a neurogenic agent.

### 2. DESCRIPTION OF THE RELATED ART

Most antidepressants are thought to work by increasing the levels of monoamines available for post-synaptic receptors. Examples of classes of agents working apparently by the "monoaminergic hypothesis of depression" include the selective serotonin uptake inhibitors (SSRIs) like fluoxetine, the mixed noradrenaline/serotonin transporter blockers like tricyclic agent imipramine and noradrenaline uptake inhibitors like desipramine. The antidepressant-induced increase in intraneuronal biogenic amines occurs quite rapidly. However, the antidepressant-induced improvement in clinical behavior requires weeks of daily administration.

One hypothesis that may account for the slow-onset of the antidepressants' therapeutic activity is that they work by promoting hippocampal neurogenesis. It is expected that neurogenesis would require a number of weeks for stem cells to divide, differentiate, migrate and establish connections with post-synaptic neurons. The neurogenesis theory of depression then postulates that antidepressant effect is brought about by structural changes in the hippocampal circuitry contributed by newly generated neurons stimulated by antidepressants (Malberg et al., 2000; Czeh et al, 2001; Santarelli et al, 2003).

The neurogenic theory of depression, though not conclusive, has strong supportive data including the finding that neurogenesis is actually requisite for antidepressant behavioral improvement in the novelty suppressed feeding model (Santarelli et al., 2003). A therapeutic benefit from hippocampal neurogenesis is further supported by the finding of hippocampal atrophy in depression, where MRI imaging studies identified a reduction in the right and the left hippocampal volumes in individuals with major depression (Sheline et al., 1996; Bremner et al., 2000; Mervaala et al., 2000). Long standing works also suggest a strong relationship between glucocorticoid dysregulation or glucocorticoid hypersecretion in stress and depression, such that the hippocampal volume loss might be considered a consequence of glucocorticoid-induced hippocampal neuronal loss (Sheline et al., 1996; Lucassen et al., 2001; Lee et al., 2002 (review)). Furthermore, in studies which involved the administration of a chronic stress to animals, both hippocampal volume changes and reduction in neurogenesis were observed, and these events were both reversed by chronic antidepressant administration (Czeh et al., 2001; Pham et al., 2003), further illustrating the strong association between depression, stress and neurogenesis. The association comes full circle, since agents or conditions that promote a reduction in neurogenesis also appear as causative agents/events in depression, specifically glucocorticoid (Sapolsky, 2000), and depletion of serotonin (Brezun and Daszuta, 1999). Kempermann and Kronenberg (2003), though acknowledging the validity of the hippocampal neurogenesis theory of depression, suggest that this hypothesis needs to be looked at in the context of a larger model of cellular, plasticity, which elucidates how antidepressants induce nascent neurons of unknown phenotype to survive and produce viable circuits.

Neurogenesis can be characterized as three successive stages: proliferation of endogenous stem cells and precursors, differentiation into neurons and neuron maturation with formation of viable synaptic connections (plasticity). By taking into account all stages of neurogenesis, then the hippocampal volume loss in depression could potentially be caused by 1) inhibition of the endogenous hippocampal stem cell proliferation in the dentate gyrus, 2) inhibition of differentiation and dendrite development and 3) by loss of neurons (apoptosis) and their dendritic structure. Though apoptosis is observed in depression, hippocampal apoptosis as measured by DNA fragmentation from depressed patients appears to play only a minor role in the volume loss (Lucassen et al., 2001). In an animal model of acute stress or in normal animals receiving exogenous corticosterone the stress did cause a reduction in synaptic plasticity in the hippocampus (Xu et al., 1998). Chronic administration of the tricyclic antidepressant, imipramine partially reversed the loss in LTP in socially stressed, depressive-like animals (Von Frijtag et al., 2001) suggesting imipramine effects on the plasticity phase of neurogenesis. In another animal model of depression, presenting neurogenesis loss and hippocampal volume loss, stressed animals that chronically received the antidepressant, tianeptine, showed similar numbers of dividing cells as control animals (no social stress) a measure of proliferation (Czeh et al., 2001). In an experiment looking at association of antidepressants and neurogenesis in normal adult rats, the antidepressant, fluoxetine required chronic administration to cause proliferation of cells in dentate gyrus (24 hrs post treatment), but there was considerable loss of nascent cells whether in the presence or absence of fluoxetine treatment, where fluoxetine provided no observed differentiation or survival benefit (Malberg et al., 2000). Results on different neurogenic intervention points by known antidepressants suggest that novel neurogenic agents that intervene at different points in the neurogenesis pathway, could result in potentially diverse therapeutic effects on depression.

These points of intervention can be studied and the target elucidated for novel antidepressant candidates through in vitro assays. Since adult stem cell proliferation and neurogenesis is observed in adult vertebrates in hippocampal dentate gyrus (Gould et al., 2001; Eriksson et al., 1998), we can use multi-potential hippocampal stem cells to screen agents in vitro for neurogenic activity.

Interestingly, chronic administration of either the antidepressant fluoxetine, an SSRI or the antidepressant rolipram, a phosphodiesterase IV inhibitor, promoted neurogenesis in normal animals (Malberg et al., 2000; Nakagawa et al., 2002). One might conclude from these results that any agent that promotes neurogenesis will generate a behavioral benefit in depression, unrelated to the agents mechanism-of-action or possibly there is a common pathway where both drug actions overlap. D'Sa and Duman (2002) suggest a scheme whereby the phosphorylation and activation of CREB and the subsequent expression of BDNF are central to the induction of neurogenesis, that culminates in antidepressant behavior. CREB phosphorylation is increased in animals administered rolipram chronically (Nakagawa et al., 2002) and antidepressants that either increase Ca2+/CaM-kinases or cAMP could cause the phosphorylation of CREB in the nucleus (reviewed by D'sa and Duman 2002). They further suggest that the phosphorylated CREB then binds to CRE binding site to promote the expression of BDNF and bcl-2, that appear critical to cell survival and plasticity. Proof of neurotrophic factor BDNF's involvement in depression comes from studies showing that antidepressants and electroconvulsive shock both caused an increase in BDNF levels (Nibuya et al., 1996) and that intrahippocampal injection of BDNF had antidepressant activity in two models of depression (Shirayama et al., 2002).

If neurogenesis is critical for antidepressant activity is it also sufficient and is the mechanism by which the neurogenesis occurs or timing of neurogenesis also critical to the therapeutic activity? We can try to answer these questions using novel agents developed through screening paradigms that identify agents that promote the proliferation and differentiation of endogenous hippocampal stem cells to neurons in vivo if they will be effective antidepressants. Since we have completed the screening of 10,000 small molecule compounds in in vitro models of neurogenesis and shown that our in vitro screen is predictive of in vivo neurogenic efficacy, we can then test these orally available agents, that promote in vivo neurogenesis, in models of depression. Rolipram, an antidepressant that works by increasing cAMP levels and is neurogenic in animals (Nakagawa et al., 2002) was effective in our primary in vitro neurogenesis screen. This suggests that our primary in vitro screen would include those agents that might promote neurogenesis by targeting the cAMP/pCREB/BDNF pathway. This does not necessarily exclude all other neurogenesis mechanisms for our NSI compounds. If the target of these neurogenic agents are important for behavioral activity where three separate chemically diverse classes showed in vitro assay efficacy differences and that the mechanism for all does not overlap at the point of CREB phosphorylation and BDNF expression then we might expect very different effects on behavioral activities in depression models.

Neuropathology associated with key cognitive regions of the Alzheimer's diseased brain have led to therapeutic strategies that address the neuronal loss, in the hopes of reducing the cognitive decline. One strategy enlists trophic agents, that regulate neuronal function and survival, as AD therapeutics (see Peterson and Gage, 1999). Problems with systemic administration, side effects and locating trophic-sensitive neurons generated few clinical successes with these therapies. One AD therapeutic, AIT-082, promotes memory enhancement in AD individuals potentially by stimulating endogenous trophic factors (Ritzman and Glasky, 1999; Rathbone et al., 1999). So the use of agents to promote increased survival and function of the remaining available neurons appears to have some therapeutic value.

Hippocampus is one of the main brain regions where neurogenesis in adult brain has been documented across several vertebrate species, including monkeys and humans (e.g., Gould et al., 2001; Eriksson et al., 1998). In fact, adult hippocampal neurogenesis contributes functionally to cognitive capacity. Shors et al. (2001) reported that inhibition of neurogenesis in adult rat hippocampus, in the absence of the destruction of existing neurons, caused impaired memory function. Many studies observed that degenerative conditions induced neurogenesis in mature mammalian brains, suggesting the existence of a natural repair pathway by means of neurogenesis. A focal ischemic model, reversible photothrombic ring stroke, caused increased neurogenesis in rat cortex by 3-6% (Gu et al., 2000). Seizures induced by electroconvulsive shock in adult rats increased neurogenesis in dentate gyrus of hippocampus (Scott et al, 2000; Madsen et al, 2000). Also, rats gamma-irradiated to kill mitotic cells in the CNS showed reduced numbers of nascent neurons and reduced LTP in slice cultures. These observations highlight the likelihood that a cellular mechanism for neurogenesis within adult human CNS, especially in hippocampus, does exist both as a normal physiological process and as a self-repairing pathway.

In adult neurogenesis a decline due to aging is observed (Kuhn et al., 1996), though proof that this age-dependent decline in neurogenesis causes cognitive impairment is still debated. Considerable evidence does exist, indicating that increased neurogenesis reduces age-associated cognitive decline. This is most dramatically observed with the transplantation of human stem cells into aged rats initiating improved water maze learning and retention (Qu et al., 2001). Other data suggests that induction of neurogenesis by diet restriction (Lee et al., 2000) exercise (van Praag et al., 1999) or growth factor addition (Lichtenwalner et al., 2001) improves learning and memory in adult or aged rats. A number of other inducers of neurogenesis have been identified, including anti-depressants (Malberg et al., 2000; Czeh et al., 2001), and nitric oxide donors (Zhang et al., 2001) suggesting the usefulness of neurogenic agents for other diseases presenting cognitive-deficits, such as stroke and depression. A small molecule that induces hippocampal neurogenesis that is blood brain barrier penetrable would allow for a potentially novel oral therapeutic for Alzheimer's disease.

Other potential AD therapeutics progressing in clinical trials, target neurodegeneration in the hopes of reducing the neuronal loss and cognitive decline. Apoptotic death involving caspase pathways and DNA fragmentation has been measured in in vitro and animal models of AD and in Alzheimer's diseased brain tissue. The extent of apoptosis leading to neuronal loss is of continual debate with most agreeing it has some effect, but that other neuronal death pathways definitely play a role (see Behl, 2000; Broe et al., 2001; Roth, 2001). Concern that measures of upstream caspase markers in neurons from AD tissue may not proceed to degeneration has been suggested (Raina et al, 2001). In order to screen for a neuroprotectant therapeutics it is critical, therefore, to measure more than one endpoint of neuronal death and determine at what point an agent may intervene in the death pathway(s). Behl (2000) suggested that AD pathology is most likely a mixture of apoptotic and necrotic pathways and that concentrating therapeutic discovery using only one pathway may provide inconclusive results. All hits in our neurogenesis models were tested through our secondary apoptosis/necrosis assay to screen for agents that function both as neurogenic and neuroprotective agents. These agents may improve or reverse the cognitive decline observed in MCI and AD.

### RELATED ART

Arsenijevic Y, Villemure JG, Brunet JF, Bloch JJ, Deglon N, Kostic C, Zurn A, Aebischer P. (2001). Isolation of multipotent neural precursors residing in the cortex of the adult human brain. Exp Neurol. vol 170(1):48-62.
Behl C. Apoptosis and Alzheimer's disease. (2000) J Neural Transm. Vol. 107 (11):1325-44.
Bremner, J.D., Narayan, M., Anderson, E.R., Staib, L.H., Miller, H.L. Charney, D.S. (2000). Hippocampal volume reduction in major depression. Am.J. Psychiatry vol 157(1):115-118.
Brezun, JM and Daszuta, A. (1999). Depletion in serotonin decreases neurogenesis in the dentate gyrus and the subventricular zone of adult rats. Neuroscience vol 89(4):999-1002.
Broe, M, Shepherd, CE, Milward, EA, and Halliday, GM. (2001) Relationship between DNA fragmentation, morphological changes and neuronal loss in Alzheimer's disease and dementia with Lewy bodies. Acta Neuropathol. (Berl) Vol. 101(6):616-624.
Calof AL, Chikaraishi DM. (1989). Analysis of neurogenesis in a mammalian neuroepithelium: proliferation and differentiation of an olfactory neuron precursor in vitro. Neuron. 3(1):115-27.
Cameron HA, Hazel TG, McKay RD. (1998). Regulation of neurogenesis by growth factors and neurotransmitters. J Neurobiol. vol 36(2):287-306.
Coon HG, Curcio F, Sakaguchi K, Brandi ML, Swerdlow RD. (1989). Cell cultures of neuroblasts from rat olfactory epithelium that show odorant responses. Proc Natl Acad Sci U S A. vol 86(5):1703-7.
Coppell, A.L., Pei, Q., Zetterstrom, T.S. (2003) Biphasic change in BDNF gene expression following antidepressant drug treatment. Neuropharmcaology vol 44(7):903-910.
Czeh, B., Michaelis, T., Watanabe, T., Frahm, J., de Biurrun, G., van Kampen, M., Bartolomucci, A., and Fuchs E. (2001). Stress-induced changes in cerebral metabolites, hippocampal volume, and cell proliferation are prevented by antidepressant treatment with tianepine. PNAS Vol. 98 (22): 12796-12801.
D'Sa, C., and Duman, D.C. (2002). Antidepressants and neuroplasticity. Bipolar Disorders vol 4:183-194.
Eriksson PS, Perfilieva E, Bjork-Eriksson T, Alborn AM, Nordborg C, Peterson DA, Gage FH. (1998). Neurogenesis in the adult human hippocampus. Nat Med. vol 4 (11):1313-7.
Falk A, Frisen J. (2002). Amphiregulin is a mitogen for adult neural stem cells. J Neurosci Res. vol 69(6):757-62.
Feron F, Mackay-Sim A, Andrieu JL, Matthaei KI, Holley A, Sicard G. (1999) Stress induces neurogenesis in non-neuronal cell cultures of adult olfactory epithelium. Neuroscience. vol 88(2):571-83.
Goldman SA, Zaremba A, Niedzwiecki D.(1992). In vitro neurogenesis by neuronal precursor cells derived from the adult songbird brain. J Neurosci. 12(7):2532-41.
Gould E, Vail N, Wagers M, Gross CG. (2001) Inaugural Article: Adult-generated hippocampal and neocortical neurons in macaques have a transient existence. Proc. Natl. Acad. Sci. USA. vol 98(19):10910-10917.
Gu W, Brannstrom T, Wester P. (2000) Cortical neurogenesis in adult rats after reversible photothrombotic stroke. J Cereb Blood Flow Metab Vol. 20(8):1166-1173.
Hauser KF, Houdi AA, Turbek CS, Elde RP, Maxson W 3rd. (2000). Opioids intrinsically inhibit the genesis of mouse cerebellar granule neuron precursors in vitro: differential impact of mu and delta receptor activation on proliferation and neurite elongation. Eur J Neurosci. vol 12(4):1281-93.
Jelitai M, Schlett K, Varju P, Eisel U, Madarasz E. (2002) Regulated appearance of NMDA receptor subunits and channel functions during in vitro neuronal differentiation. J Neurobiol. vol 51(1):54-65.
Jin K, Mao XO, Sun Y, Xie L, Greenberg DA. (2002). Stem cell factor stimulates neurogenesis in vitro and in vivo. J Clin Invest. vol 110(3):311-9.
Jin K, Zhu Y, Sun Y, Mao XO, Xie L, Greenberg DA. (2002). Vascular endothelial growth factor (VEGF) stimulates neurogenesis in vitro and in vivo. Proc Natl Acad Sci U S A. vol 99(18):11946-50.
Kehl LJ, Fairbanks CA, Laughlin TM, Wilcox GL. (1997). Neurogenesis in postnatal rat spinal cord: a study in primary culture. Science. vol 276(5312):586-9.
Kempermann, G. and Kronenberg, G. (2003) Depressed new neurons-adult hippocampal neurogenesis and a cellular plasticity hypothesis of major depression. Biol Psychiatry vol 54 (5) :499-503.
Kempermann, G. and Gage, FH (2002). Genetic determinants of adult hippocampal neurogenesis correlate with acquisition, but not probe trial performance, in the water maze task. Eur J of Neurosci, 16, 129-36.
Kuhn, H.G., Dickenson-Anson, H. and Gage, F.H. (1996) Neurogenesis in the dentate gyrus of the adult rat: age-related decrease of neuronal progeneitor proliferation. J. Neurosci. vol 16 (6), pp2027-33.
Lee, A.L., Ogle, W.O., Sapolsky, R.M. (2002). Stress and depression:possible links to neurons death in the hippocampus. Bipolar Disord. vol 4(2):117-128.
Lee,J., Duan, W., Long, J.M., Ingram, D.K., and Mattson, M.P. (2000) Dietary restriction increases the number of newly generated neural cells, and induces BDNF expression, in the dentate gyrus of rats. J. Mol. Neurosci.. vol.15 (2), pp 99-108.
Lichtenwalner, R.J., Forbes, M.E., Bennett, S.A., Lynch, C.D., Sonntag, W.E., and Riddle, D.R. (2001) Intracerebroventricular infusion of insulin-like growth factor-1 ameliorates the age-related decline in hippocampal neurogenesis. Neuroscience vol. 107 (4), pp603-613.
Lucassen, P.J., Muller, M.B., Holsboer, F., Bauer, J., Holtrop, A., Wouda, J., Hoogendijk, W.J., DeKloet, E.R., Swaab, D.F. (2001). Hippocampal apoptosis in major depression is a minor event and absent from subareas at risk for glucocorticoid overexposure. Am.J. Pathol. vol 158(2):453-468.
Ma W, Maric D, Li BS, Hu Q, Andreadis JD, Grant GM, Liu QY, Shaffer KM, Chang YH, Zhang L, Pancrazio JJ, Pant HC, Stenger DA, Barker JL. (2000). Acetylcholine stimulates cortical precursor cell proliferation in vitro via muscarinic receptor activation and MAP kinase phosphorylation. Eur J Neurosci. vol 12(4):1227-40.
Madsen TM, Treschow A, Bengzon J, Bolwig TG, Lindvall O, Tingstrom A. (2000) Increased neurogenesis in a model of electroconvulsive therapy. Biol Psychiatry Vol. 47(12):1043-1049.
Malberg, J.E., Eisch, A.J., Nestler, E.J., and Duman, R.S. (2000). Chronic antidepressant treatment increases neurogenesis in adult rat hippocampus. J. Neurosci.. vol. 20 (29):9104-9110.
Marin N, Romero B., Bosch-Morell F., Llansola M., Felipo V., Roma J.,and Romero F.J. (2000) □-amyloid-induced activation of caspase-3 in primary cultures of rat neurons. Mech. Ageing and Devl. Vol. 119:63-67.
Mayo W, LeMoal M, Abrous DN. (2001) Pregnenolone sulfate and aging of cognitive functions: behavioral, neurochemical, and morphological investigations. Horm Behav Vol. vol 40(2):215-217.
Mervaala, E., Fohr, J., Kononen, M., Valkonen-Korhonen, M., Vainio, P., Partanen, K., Partanen, J., Tiihonen, J., Viinamaki, H., Karjalainen, A.K., Lehtonen, J. (2000). Quantitative MRI of the hippocampus and amygdala in severe depression. Psychol. Med. vol 30(1):117-125.
Murrell W, Bushell GR, Livesey J, McGrath J, MacDonald KP, Bates PR, Mackay-Sim A. (1996). Neurogenesis in adult human. Neuroreport vol 26;7(6):1189-94.
Nakagawa, S., Kim, J-E, Le R., Malberg, J.E., Chen, J., Steffen, C., Zhang, Y-J., Nestler, E.J., Duman, R.S. (2002). regulation of neurogenesis in adult mouse hippocampus by cAMP and the cAMP reponse element-binding protein. J. Neurosci. vol 22(9):3673-3682.
Nibuya,, M., Nestker,E,J., Duman, R.S. (1996). Chronic antidepressant administration increases the expression of cAMP response element binding protein (CREB) in rat hippocampus. Neurosci. Lett. vol 267:81-84.
Nestler,E.J., Barrot, M., DiLeone, R.J., Eisch, A., Gold, S.J., and Monteggia, L.M. (2002). Neurobiology of Depression. Neuron, vol 34:13-25.
Palmer TD, Markakis EA, Willhoite AR, Safar F, Gage FH. (1999) Fibroblast growth factor-2 activates a latent neurogenic program in neural stem cells from diverse regions of the adult CNS. J Neurosci. vol 19(19):8487-97.
Peterson D.A. and Gage F.H. (1999) Trophic factor therapy for neuronal death. In: Alzheimer Disease, Terry, Katzman, Bick, Sisodia eds 2nd edition.
Pham, K., Nacher, J., Hof, PR., McEwen, B.S. (2003). Repeated restraint stress suppresses neurogenesis and induces biphasic PSA-NCAM expression in the adult dentate gyrus. Eur. J. Neurosci. vol 17(2):879-886.
Pincus DW, Harrison-Restelli C, Barry J, Goodman RR, Fraser RA, Nedergaard M, Goldman SA. (1997). In vitro neurogenesis by adult human epileptic temporal neocortex. Clin Neurosurg. vol 44:17-25.
Qu, T, Brannan, C.L., Kim, H.M., and Sugaya, K. (2001) Human neural stem cells improve cognitive function of aged brain. Neuroreport vol. 12 (6), pp. 1127-32.
Raina, AK, Hochman A., Zhu, X., Rottkamp, C.A., Nunomura, A., Siedlak, S.L., Boux, H., Castellani, R.J., Perry, G., Smith, M.A. (2001) Abortive apoptosis in Alzheimer's disease. Acta Neuropahtol (Berl) Vol. 101(4):305-310.
Rathbone MP, Middlemiss PJ, Gysbers JW, Andrew C, Herman MA, Reed JK, Ciccarelli R, Di Iorio P, and Caciagli F. (1999) Trophic effects of purines in neurons and glial cells. Prog. Neurobiol. Vol. 59(6):663-90.
Ritzman R, Glasky AJ. (1997) Psychopharmacological actions of AIT-082. Soc. Neurosci. Abs. Vol. 23:1896. Rozental R, Mehler MF, Morales M, Andrade-Rozental AF, Kessler JA, Spray DC. (1995). Differentiation of hippocampal progenitor cells in vitro: temporal expression of intercellular coupling and voltage- and ligand-gated responses. Dev Biol. 167(1):350-62.
Roth, K.A. (2001) Caspases, apoptosis, and Alzheimer disease: causation, correlation, and confusion. J. Neuropathol. Exp. Neurol. Vol. 60(9):829-838.
Roy NS, Wang S, Jiang L, Kang J, Benraiss A, Harrison-Restelli C, Fraser RA,Couldwell WT, Kawaguchi A, Okano H, Nedergaard M, Goldman SA. (2000) In vitro neurogenesis by progenitor cells isolated from the adult human hippocampus. Nat Med. vol 6(3):271-7.
Santarelli, L., Saxe, M., Gross, C., Surget, A., Battaglia, F., Dulawa, S., Weisstaub, N., Lee, J., Duman, R., Arancio, O., Belzung, C., and Hen, R. (2003). Requirement of hippocampal neurogenesis for the behavioral effects of antidepressants. Science vol 301:805-809.
Sapolsky, R.M. (2000). The possibility of neurotoxicity in the hippocampus in major depression: a primer on neuron death. Biol. Psychiatry vol 48(8):775-765.
Satoh M, Yoshida T. (1997). Promotion of neurogenesis in mouse olfactory neuronal progenitor cells by leukemia inhibitory factor in vitro. Neurosci Lett. vol 225(3):165-8.
Scott BW, Wojtowicz JM, Burnham WM. (2000) Neurogenesis in the dentate gyrus of the rat following electroconvulsive shock seizures. Exp Neurol Vol. 165(2):231-236.
Seaberg RM, van der Kooy. (2002) Adult rodent neurogenic regions: the ventricular subependyma contains neural stem cells, but the dentate gyrus contains restricted progenitors. J Neurosci. vol 22(5):1784-93.
Sheline, Y.I., Wang, P.W., Gado, M.H., Csernansky, J.G., Vannier, M.W. (1996). Hippocampal atrophy in recurrent major depression. Proc. Natl. Acad. Sci. USA vol 93:3908-3913.
Shingo T, Sorokan ST, Shimazaki T, Weiss S. (2001). Erythropoietin regulates the in vitro and in vivo production of neuronal progenitors by mammalian forebrain neural stem cells. J Neurosci. vol 21(24):9733-43.
Shirayama, Y., Chen, A.C.-H., Nakagawa, S., Russell, D.S., Duman, R.S. (2002). Brain-derived neurotrophic factor produces antidepressant effects in behavioral models of depression. J. Neurosci. vol 22(8):3251-3261.
Shors TJ, Miesegaes G, Beylin A, Zhao M, Rydel T, Gould E. (2001) Neurogenesis in the adult is involved in the formation of trace memories. Nature vol 410(6826):372-376. Shou J, Rim PC, Calof AL. (1999). BMPs inhibit neurogenesis by a mechanism involving degradation of a transcription factor. Nat Neurosci. 2(4):301-3.
Takahashi J, Palmer TD, Gage FH. (1999). Retinoic acid and neurotrophins collaborate to regulate neurogenesis in adult-derived neural stem cell cultures. J Neurobiol. vol 38(1):65-81.
Taupin P, Ray J, Fischer WH, Suhr ST, Hakansson K, Grubb A, Gage FH. (2000). FGF-2-responsive neural stem cell proliferation requires CCg, a novel autocrine/paracrine cofactor. Neuron. vol 28(2):385-97.
Von Frijtag, J.C., Kamal, A., Reijmers, L.G., Schrama, L.H., van den Bos, R., Spruijt, B.M. (2001). Chronic imipramine treatment partially reverses the lnog-term changes of hipocampal synaptic plasticity in socially stressed rats. Neurosci. Lett. vol 309(3):153-156.
Wohl CA, Weiss S. (1998). Retinoic acid enhances neuronal proliferation and astroglial differentiation in cultures of CNS stem cell-derived precursors. J Neurobiol. vol 5;37(2):281-90.
Xu, L., Holscher, C., Anwyl, R., Rowan, M.J. (1998). Glucocorticoid receptor and protein/RNA synthesis-dependent mechanisms underlie the control of synaptic plasticity by stress. PNAS USA. vol 95:3204-3208.
Zhang, R., Zhang, L., Zhang, Z., Wang, Y., Lu, M., Lapointe, M., and Chopp, M. (2001) A nitric oxide donor induces neurogenesis and reduces functional deficits after stroke in rats. Ann. Neurol. vol. 50 (5), pp 602-11.

WO 01/88104 refers to populations of neural progenitor cells, differentiated neurons, glial cells, and astrocytes obtained by culturing stem cell populations under conditions initiating differentiation to achieve the neural progenitor population. The progenitors may be further differentiated to different neural phenotypes. The differentiated cell populations or the neural progenitors may be employed in drug screening and the treatment of neurological disorders. WO 02/058736 pertains to inducing neurogenesis by administering particular compounds, such as leteprinim potassium, inducing neurogenesis, wherein neurogenesis includes proliferation of neural stem and progenitor cells, and differentiation into neurons.

### SUMMARY OF THE INVENTION

A neurogenic drug is an agent that enhances the process of generating new neurons (neurogenesis). Recent studies indicate that neurogenesis occurs in the adult human brains under normal as well as under degenerative conditions and that such adult-generated neurons do contribute functionally to the brain physiology such as learning and memory. These observations highlight the likelihood that a cellular mechanism for neurogenesis within adult human CNS, especially in hippocampus, does exist both as a normal physiological pathway and as a self-repairing pathway. What is lacking and contributes to permanent damage may be (1) the volume/persistence of neurogenesis and/or (2) the survival/maturation of the new neurons. The objective of the neurogenesis screen as described here is to discover a compound that will significantly boost either of these processes.

Many neurological diseases, including Alzheimer's disease, mild cognitive impairment, dementia, age-related cognitive decline, stroke, traumatic brain injury, spinal cord injury and the like are neurodegenerative conditions. Neuropsychiatric diseases including depression, anxiety, schizophrenia and the like also show nerve cell dysfunction leading to cognitive, behavioral, and mood disorders. A neurogenic drug would be beneficial for countering and treating these diseases.

The present invention discloses a method of identifying a neurogenic agent, comprising:
providing a stable human neural progenitor cell line, wherein
   (i) the cell line is derived from an area of the central nervous system,
   (ii) the cell line has been expanded for at least ten cell doublings, and
   (iii) the cell line generates both neurons and glia upon differentiation in the absence of mitogen;
plating cells of said cell line into a culture vessel with a serum-free, mitogen-free medium; contacting the cells with a test agent;
incubating the cells in the presence of the test agent for at least seven days; and
after said 7 days, assessing the number of neurons by immunostaining with antibodies against neurons and the number of respiring cells using a fluorescent dye as a measure of proliferation, wherein an increase of at least 30% in proliferation over vehicle control and an increase of at least 10% of neuron numbers over vehicle control identifies the test compound as a neurogenic agent, wherein the test agent
   is a fused imidazole, as described in Structure Formula 1, or
   is an aminopyrimidine, as described in Structure Formula 2; or
   is a nicotinamide, as described in Structure Formula 3; or
   is an aminomethyl phenoxypiperidine, as described in Structure Formula 4; or
   is an aryloxypiperidine, as described in Structure Formula 5.
Such drug will serve to prevent or treat neurodegenerative and neuropsychiatric disorders by promoting the birth of new neuron endogenously within the nervous system by administering the compounds of the present invention into the patient. This may involve delivery of the agents alone or together with transplanted stem cells or progenitor cells.

Using the method herein, compounds of the type, Fused Imidazoles, Aminopyrimidines, Nicotinamides, Aminomethyl Phenoxypiperidines and Aryloxypiperidines may be evaluated for their ability to promote neurogenesis by proliferation/differentiation of human hippocampal multipotent stem/progenitor cells and neuronal progenitors.

The invention further pertains to an assay for identifying a neurogenic agent, said assay comprising:
a stable human neural progenitor cell line, wherein
   (i) the cell line is derived from an area of the central nervous system,
   (ii) the cell line has been expanded for at least ten cell doublings, and
   (iii) the cell line differentiates into both neurons and glia upon differentiation in the absence of mitogen;
an assay plate pre-coated with extracellular matrix proteins, wherein undifferentiated neural progenitor cells of said cell line are cultured in a serum-free, mitogen free medium;
a test agent; and
antibodies immunoreactive with neurons for assessing neuron number and a fluorescent dye for assessing proliferation, wherein the test agent
is a fused imidazole, as described in Structure Formula 1, or
is an aminopyrimidine, as described in Structure Formula 2; or
is a nicotinamide, as described in Structure Formula 3; or
is an aminomethyl phenoxypiperidine, as described in Structure Formula 4; or
is an aryloxypiperidine, as described in Structure Formula 5.

### BRIEF DESCRIPTION OF THE DRAWING

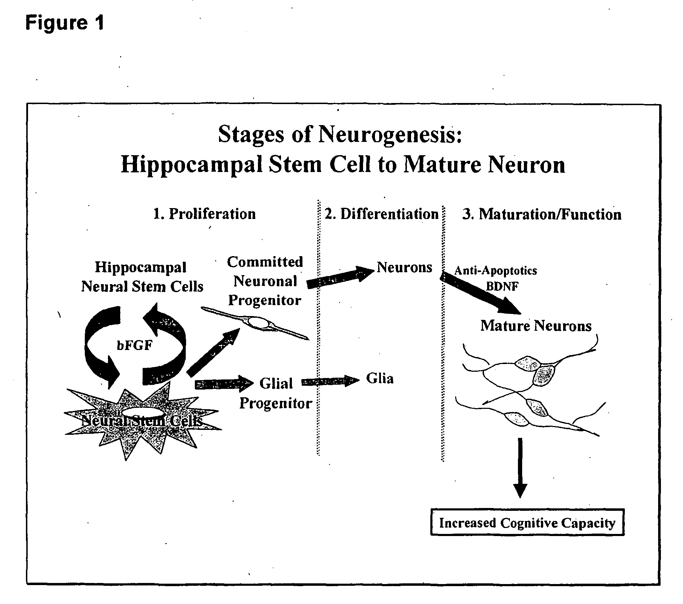

Figure 1. Schematic description of neurogenesis processes captured in the assay and different potential sites of a neurogenic drug action.

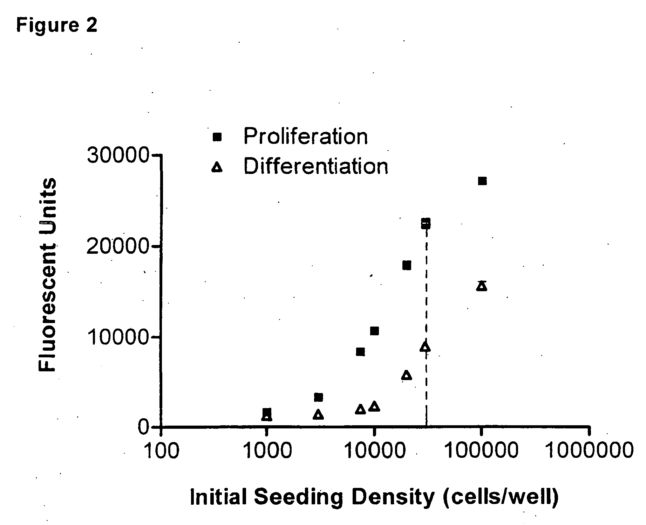

Figure 2. Detection of changes in cell number by Alamar Blue dye. Alamar Blue, a fluorescent dye, is used as an indicator of metabolic respiration to determine optimum plating density. Results at an initial plating density of 30,000 cells/well suggest a large difference in cell number on removal of mitogen from the N2b media (differentiation) versus N2b with mitogen (proliferation) conditions. This figure only describes total cellular activity, further markers are required to determine what cell types (e.g. neuronal, glial) are observed under differentiating media.

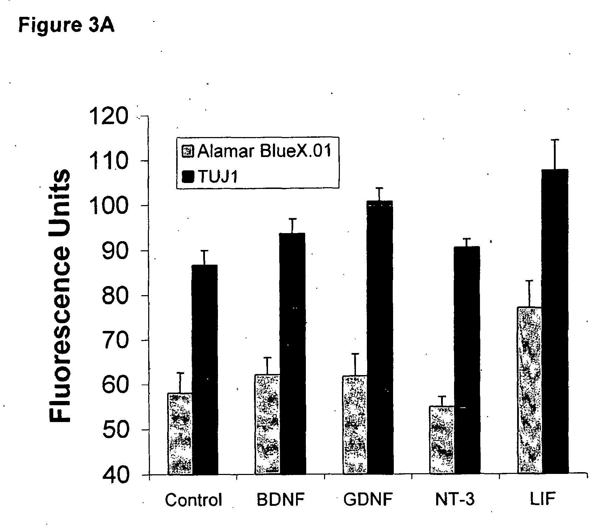

Figure 3A. Influence of known growth factors on proliferation and neurogenesis relative to control. Hippocampal progenitor cells were treated for seven days with differentiation media (without mitogen) in the presence or absence of 20 ng/ml of growth factor dosed every other day. Plates were treated with Alamar Blue as described in Methods, then fixed and stained with antibody (TuJ1) against type III beta-tubulin (neuronal marker). The 96-well plate was read in a fluorescent plate reader. Bars represent the Mean+SD from 4 wells per treatment.

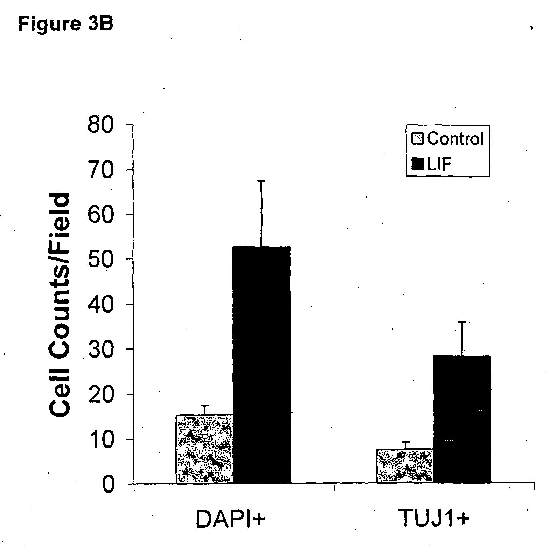

Figure 3B. LIF effects on hippocampal cell proliferation and neurogenesis by manual cell counting. Hippocampal progenitor cells were treated for seven days with differentiation media (without mitogen) in the presence or absence of 20 ng/ml LIF. Three fields were analyzed per well for total number of cells (DAPI positive nuclei) and for total number of neurons (TUJI positive cells). Bars represent the Mean+SD from 4 wells per treatment. The percentage of neurons calculated for each treatment are as follows: 48.5 + 6.3% for controls and 53.6+1.15 for LIF. The non-TUJ1 positive cells are mainly astrocytic (GFAP+).

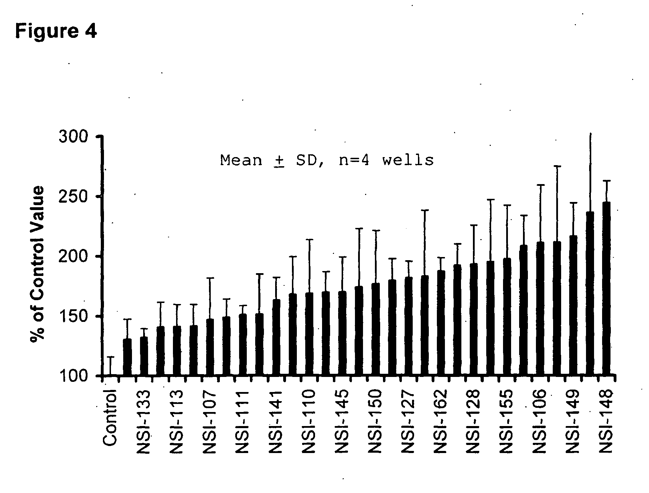

Figure 4. Examples of proliferation profile of compounds selected from primary screening. Proliferation was measured after compound treatment for 7 days by Alamar Blue staining of live cells per well. Shown are relative values over the vehicle control.

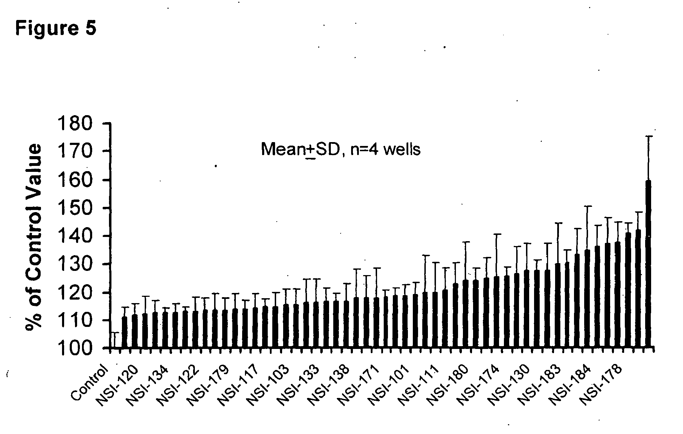

Figure 5. Example of neurogenesis profile of compounds selected from primary screening. After 7 days of compound treatment, the ratio of neuron number (TuJ1 stained) to the total nuclei number (Hoechst stained) was determined. Shown are the relative ratio of neurori:total cells for each compound over the vehicle control in percentage. Typical ratio for vehicle control is 40-50% neurons. The ratio can change by either increased differentiation of the cells to neurons, decreased proliferation of astrocytes, or increased proliferation of neuronal progenitors.

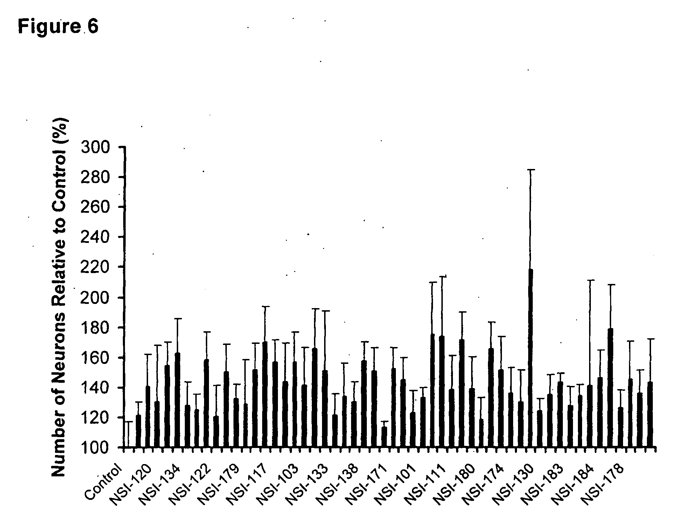

Figure 6. Examples of neurogenesis profile of compounds selected from primary screening. After 7 days of compound treatment, the cells were stained with TuJ1 for neurons. The absolute number of TuJ1+ neurons per area was quantified and expressed as a relative value to the vehicle treated control.

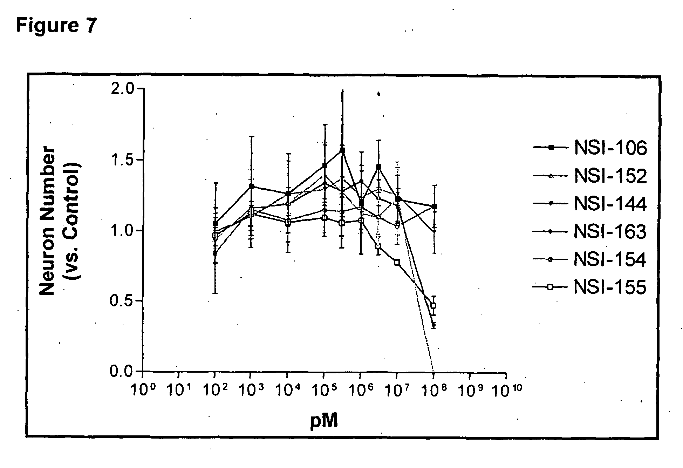

Figure 7. Dose-dependent increase in neuron number. Differentiating human hippocampal progenitor cells were treated for 7 days with varying concentrations of "primary hits". Subsequently, the cells were fixed, stained with TuJ1, and positive cells were quantified by an automated cell counter. Shown are the number of neurons after each treatment normalized against the vehicle control (0 microM =1.0) .

### DETAILED DESCRIPTION OF THE INVENTION

### 1. A Stable Cell Line of Neural Progenitors

A screening of a large number of unknown agent (e.g., protein factors, peptides, nucleic acids, natural compounds, or synthetic compounds) for discovering a candidate drug involves repeating the same test for several hundreds to several million times. This requires a great deal of reproducibility from the test. In order to obtain such reproducibility for neurogenesis assay, we have created stable cell lines of neural progenitors, which upon differentiation generate reproducible quantities of neurons. A multipotent neural stem/progenitor cell line derived from human hippocampus may be used. Cell lines derived from other CNS areas, including dentate gyrus of an adult brain, can also substitute. A neural progenitor population derived as a stable cell line from partial differentiation of embryonic stem cells can also be used. For this purpose, a cell line is defined as a population of cells having been expanded for at least 10 cell-doublings.

Cell lines that are genetically engineered to enhance the cells' mitotic capacity can also be used. The genetic modification may consist of over-expression of functional c-myc protein intracellularly under a conditional activation system such as c-myc protein fused to a ligand-binding domain of an estrogen receptor. Cell lines that are not genetically engineered are preferred and can also be used.

In a preferred embodiment, a progenitor population that upon differentiation generates both neurons and glia in a single culture has been used. Presence of glia, either astrocytes and/or oligodendrocytes or their precursors, are required to promote physiological maturation of nascent neurons born from their precursors in culture.

In a preferred embodiment, differentiation of the progenitors is initiated by withdrawing the mitogen from the culture. Serum as well as other growth-promoting factors should be avoided from the differentiating culture since they will significantly affect the reproducibility and interfere with the neurogenesis assay.

### 2. Preparation of Assay Plate

Neural stem/progenitor cells differentiate spontaneously in the absence of a mitogen. Undifferentiated mitotic cells are harvested by enzyme treatment to remove residual mitogen, in the preferred embodiment, basic fibroblast growth factor (bFGF). The collected cells are seeded into appropriate plates (standard 96-well or 384-well) pre-coated with the usual extra cellular matrix proteins (poly-D-lysine and fibronectin, for example) for attachment of the cells. The initial seeding density can be within the range of about 2,000-125,000 cells per well of a 96-well plate. The preferred density is 40,000 cells per well of a 96-well plate, which has been optimized for best signal-to-noise ratio. Too low cell density retards the initiation of differentiation and results in poor plating efficiency, which interferes with the assay. Too high cell density leads to inhibition of neurogenesis due to cell-cell contact and paracrine factors, which also interferes with the assay. The actual cell number can be proportionally decreased or increased depending upon the surface area of the culture substrate used. For example, for a 384-well plate, which has approximately 1/4 of the surface area of a 96-well plate, the initial seeding density should be decreased accordingly (1/4).

### 3. Detection of Neurogenesis

The key activity of a neurogenic drug is to increase the number of neurons generated from their precursors. A molecule can bring about such increase in the neurogenesis by a number of different mechanisms. It can act as a mitogen for the neural stem/progenitor cells and increase the progenitor's cell number, which in turn results in increased number of neurons in the culture when differentiated. Or, it can act as a neuronal specification factor by promoting the stem/progenitor cell differentiation toward neurons in the expense of glia. This will also result in increased number of neurons in the culture, but without changing the overall cell number. Or, it can act as a mitogen for committed neuronal progenitors that differentiate only into neurons. Increasing this subpopulation would also increase the final number of neurons in the culture. Or, it can act as a survival factor to rescue immature neurons from undergoing cell death during differentiation, which will result in increased neurons (Figure 1).

The assay method here captures all of these possibilities by allowing for sufficient time for these processes to unfold. In a preferred embodiment, for human neural stem/progenitor cells, the assay is continued for seven days. A minimum of three days from the onset of differentiation should be allowed for stable expression of definitive neuronal markers to appears. A sufficient time is also required for a compound action on differentiation and/or proliferation to take place to a sufficient degree to be reliably detectable. Manifestation of drug-induced changes in neuron number takes a minimum of three days for the human cells to be detectable.

The final neuron number is detected by immunostaining of the culture with antibodies against neurons and quantified by counting of the immunopositive neurons and/or by measuring the staining intensity.

### 4. Method for Measuring Neurogenesis

(1) Undifferentiated human neural stem/progenitor cells were harvested by enzyme treatment.
(2) The collected cells were seeded at 40,000 cells per well of 96-well plates pre-coated with extracellular matrix proteins (e.g., Biocoat PDL, Fisher). The seeding media is a standard serum-free, growth factor-free, basal media that supports healthy neuronal/glial survival, such as N2 without phenol red.
(3) Test agents at appropriate concentrations were added to each well on Day 0.
(4) The assay plates were incubated for 7 days, with 50% media change at every other day. On Day 2, 4, and 6 of post-plating, additional increment of the screening agents at appropriate concentrations were added to each well.
(5) On the final day of the culture (Day 7), alamar blue dye was added to each well and the cultures were further incubated for 2 hours at 37°C.
(6) The fluorescence of the oxidized dye in each well was read by a fluorescent plate reader with the following settings:
   Read Mode End Point
   Excitation 530nm, emission 590nm, cutoff 570nm The fluorescence level is proportional to the number of respiring cells in the culture and is a measure of a proliferative activity of a test agent (Figure 2).
(7) After the alamar blue assay, the cells were fixed and stained with antibodies against neuron-specific antigens according to standard procedures. Typical antigens effective were TypeIII-beta tubulin and MAP2c.
(8) The total cell number in each well was quantified by staining the cultures with a nuclear dye such as DAPI or Hoechst according to standard procedures.
(9) As a preliminary detection of positive activities, the overall immunostaining intensity in each well was read by a fluorescence plate reader. For the positive hits, more quantitative analysis was carried out by automated morphometric counting of individual cells.

### 5. Examples

Example 1. Selection of a positive control. Several neurotrophic factors--including brain-derived neurotrophic factor, glia-derived neurotrophic factor, neurotrophic factor-3, and leukemia inhibitory factor--suggested to have neurogenic properties were tested in the assay described above. Only one (leukemia inhibitory factor) was effective (Figure 3A and 3B). Thus, the assay can discriminate test agents for selectively having a neurogenic activity. The positive control utilized is leukemia inhibitory factor (LIF), a cytokine growth factor, at 20 ng/ml. The selection of LIF as the positive control is based on its properties to increase by 2-3 fold the number of neurons and glia. This effect validates both the neural stem cell system, in which, should a compound be effective in neurogenesis, the cells respond appropriately by enhanced differentiation and/or mitosis, and the assay method in which such cellular responses can be measured reproducibly and quantifiably. Example 2. Primary screening of unknown compounds.

5,628 synthetic compounds of the type Fused Imidazoles, Aminopyrimidines, Nicotinamides, Aminomethyl Phenoxypiperidines and Aryloxypiperidines are evaluated for their effect on neurogenesis according the assay method described above. From the preliminary analysis using the fluorescent plate reader, over 300 compounds to date showed initial positive activity. Those were re-analyzed by quantitative neuron counting. Among them, 30 compounds significantly increased cell number ("proliferation", Figure 4); 53 increased the number of neurons ("neurogenesis", Figure 5 & Figure 6); and 7 showed significant activity in both. The significance level was empirically set at an activity above 30% change over the vehicle control for proliferation and above 10% change for neurogenesis. A summary of the result in the compound screening is provided in Table I.

**Table I. Summary of Compound Screening**

| Primacy Screen | Hits Confirmed | Proliferation Hit | Neurogenesis Hit | Double Hit |
|---|---|---|---|---|
| 0 | 0 | 0 | 0 | 0 |
| 2,240 | 88 | 13 | 8 | 1 |
| 5,628 | >300 | 30 | 53 | 7 |

### Example 3. Dose-response profiles.

Linearity of dose-response and in vitro neurotoxicity are used to further filter down desired compounds from the primary screen. The dose-response curve measures neurogenesis over a concentration range of 100picaM to 100microM. The rationale for this is to eliminate early on those compounds with pronounced toxicity and those without a dose-dependent effect on neurogenesis. Examples of several primary hits fully analyzed for dose-response are shown in Figure 7. Significantly, most compounds exhibit a linear response over several log concentrations below ImicroM. This indicates that the assay for primary screening is reliable and that the quality of the compound library is high. Table II contains the summary of EC50 of each compound tested. On the other hand, at high concentrations (100 microM), some, but not all showed high level of neurotoxicity, indicating that analyzing dose-response curves will be discriminatory and.serve as an effective early filter.

**Table II. Activity Profile of Primary Hits in Vitro**

| Compound ID | Proliferatio n (% of Control) | Neuron Ratio (% of Control | EC50 for Neuron Number | Other Characteriz ation Of Toxicity |
|---|---|---|---|---|
| NSI-106 | 211 + 48 | 92 + 6 | 0. nM r² 0.75 | No Toxicity |
| NSI-144 | 149 + 15 | 137 + 8 | 1.0nM r² 0.54 | No Toxicity |
| NSI-152 | 174 + 49 | 112 + 4 | 0.1nM r² 0.84 | Toxic At Highest Dose |
| NSI-154 | 211 + 63 | 102 + 6 | 0.3nM r² 0.79 | No Toxicity |
| NSI-155 | 198 + 44 | 118 + 8 | 0.05nM r² 0.4 | Toxic At Highest Dose |
| NSI-163 | 208 + 25 | 120 + 11 | 1.0nM r² 0.81 | Toxic At Highest Dose |

### UTILITIES WHICH DO NOT FORM PART OF THE PRESENT INVENTION

An agent might be administered to treat a neurodegenerative disease. The neurodegenerative disease might be Alzheimer's disease, dementia, mild cognitive impairment, aged-related cognitive decline, Parkinson's disease, amyotrophic lateral sclerosis, multiple sclerosis, demyelination, stroke, spinal injuries, traumatic injuries, neuropathic pain, and the like.

The agent might the psychiatric disease may be depression, post-traumatic stress syndrome, stress, anxiety, schizophrenia, sleep deprivation, cogntive dysfunction, amnesia, and the like.

An agent might be administered by any number of routes and multipotent stem cells or differentiated multipotent stem cells might be transplanted into brain.

The structures of the formula which may be utilized in above methods:

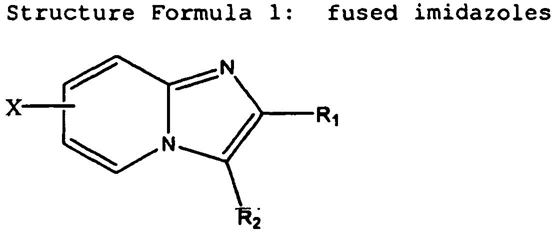

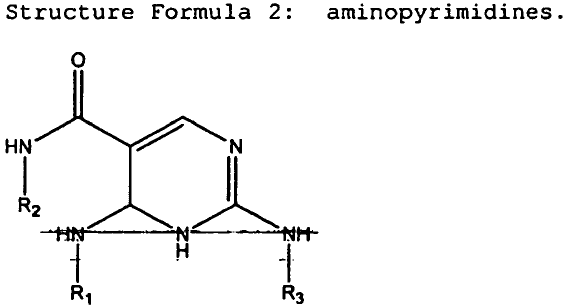

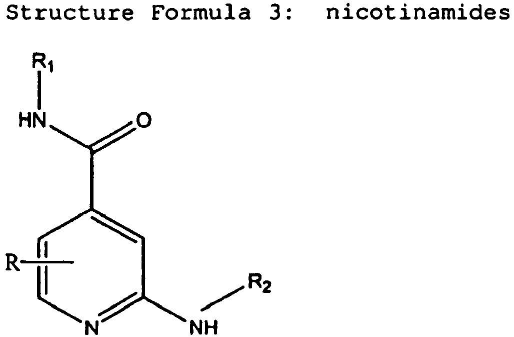

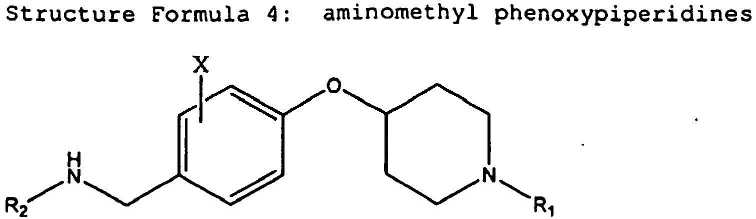

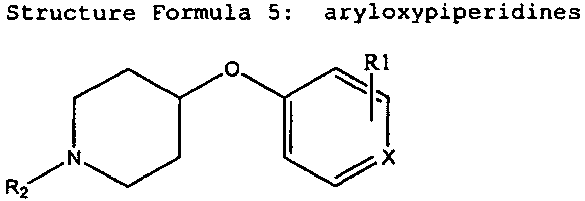

While the invention has been described in connection with what is presently considered to be the most practical and preferred embodiments, it is to be understood that the invention is not limited to the disclosed embodiments, but on the contrary is intended to cover various modifications.

Thus, it is to be understood that variations in the present invention can be made without departing from the novel aspects of this invention as defined in the claims.

## Claims

1. A method of identifying a neurogenic agent, comprising:
providing a stable human neural progenitor cell line, wherein
(i) the cell line is derived from an area of the central nervous system,
(ii) the cell line has been expanded for at least ten cell doublings, and
(iii) the cell line generates both neurons and glia upon differentiation in the absence of mitogen;
plating cells of said cell line into a culture vessel with a serum-free, mitogen-free medium;
contacting the cells with a test agent;
incubating the cells in the presence of the test agent for at least seven days; and
after said 7 days, assessing the number of neurons by immunostaining with antibodies against neurons and the number of respiring cells using a fluorescent dye as a measure of proliferation, wherein an increase of at least 30% in proliferation over vehicle control and an increase of at least 10% of neuron numbers over vehicle control identifies the test compound as a neurogenic agent, wherein the test agent
is a fused imidazole, as described in Structure Formula 1, or
is an aminopyrimidine, as described in Structure Formula 2; or
is a nicotinamide, as described in Structure Formula 3; or
is an aminomethyl phenoxypiperidine, as described in Structure Formula 4; or
is an aryloxypiperidine, as described in Structure Formula 5.

2. The method of claim 1, wherein the antibodies against neurons are immunoreactive with Type III β-tubulin, MAP2c or TUJ1.

3. The method of claim 1 or 2, wherein the florescent dye is AlamarBlue^{®}.

4. The method of any of claims 1-3, wherein cells are contacted with the test agent on days 0, 2, 4 and 6.

5. The method of any of claims 1-4, wherein said cells are plated at a density corresponding to about 40,000 cells per well of a 96 well plate.

6. The method of any of claims 1-5, which is conducted using leukemia-inhibitory factor (LIF) as a positive control.

7. The method of any of claims 1-6, wherein the area of the central nervous system is hippocampus or subventricular zone.

8. An assay for identifying a neurogenic agent, said assay comprising:
a stable human neural progenitor cell line, wherein
(i) the cell line is derived from an area of the central nervous system,
(ii) the cell line has been expanded for at least ten cell doublings, and
(iii) the cell line differentiates into both neurons and glia upon differentiation in the absence of mitogen;
an assay plate pre-coated with extracellular matrix proteins, wherein undifferentiated neural progenitor cells of said cell line are cultured in a serum-free, mitogen free medium;
a test agent; and
antibodies immunoreactive with neurons for assessing neuron number and a fluorescent dye for assessing proliferation, wherein the test agent
is a fused imidazole, as described in Structure Formula 1, or
is an aminopyrimidine, as described in Structure Formula 2; or
is a nicotinamide, as described in Structure Formula 3; or
is an aminomethyl phenoxypiperidine, as described in Structure Formula 4; or
is an aryloxypiperidine, as described in Structure Formula 5.

9. The assay of claim 8 wherein the antibodies against neurons are immunoreactive with Type III β-tubulin, MAP2c or TUJ1.

10. The assay of claim 8 or 9, wherein the florescent dye is AlamarBlue^{®}.

11. The assay of any of claims 8-10, wherein the undifferentiated neural precursor cells are plated at a density of about 40,000 cells per well in a 96-well plate.

12. The assay of any of claims 8-11, which further includes LIF as a positive control.

13. The assay of claims 9-12, wherein the area of the central nervous system is hippocampus or subventricular zone.

## Patentansprüche

1. Verfahren zum Identifizieren eines neurogenen Agenz, umfassend:
Bereitstellen einer stabilen humanen neuralen Vorläufer-Zelllinie, wobei
(i) die Zelllinie aus einem Bereich des zentralen Nervensystems abgeleitet ist,
(ii) die Zelllinie mit wenigstens zehn Verdoppelungen entwickelt wurde, und
(iii) die Zelllinie sowohl Neuronen als auch Gliazellen bei einer Differenzierung ohne Mitogen erzeugt;
Ausplattieren der Zellen der Zelllinie in einem Kulturgefäß mit einem Medium ohne Serum und ohne Mitogen;
Inkontaktbringen der Zellen mit einem Testagenz;
Inkubieren der Zellen in Gegenwart des Testagenz für mindestens sieben Tage; und
nach den sieben Tagen, Erfassen der Anzahl an Neuronen durch Immunfärben mit Antikörpern gegen Neuronen und der Anzahl an respirierenden Zellen unter Verwendung eines Fluoreszenzfarbstoffes als ein Maß für die Proliferation, wobei eine Erhöhung der Proliferation um wenigstens 30 % verglichen mit einer Trägerkontrolle und eine Erhöhung der Neuronenanzahl um wenigstens 10 % verglichen mit einer Trägerkontrolle das Testagenz als neurogenes Agenz identifiziert, wobei das Testagenz
ein fusioniertes Imidazol wie in Strukturformel 1 beschrieben ist, oder
ein Aminopyrimidin wie in Strukturformel 2 beschrieben ist, oder
ein Nikotinamid wie in Strukturformel 3 beschrieben ist, oder
ein Aminomethylphenoxypiperidin wie in Strukturformel 4 beschrieben ist, oder
ein Aryloxypiperidin wie in Strukturformel 5 beschrieben ist.

2. Verfahren nach Anspruch 1, wobei die Antikörper gegen Neuronen immunreaktiv mit Typ III β-Tubulin, MAP2c oder TUJ1 sind.

3. Verfahren nach Anspruch 1 oder 2, wobei der Fluoreszenzfarbstoff AlamarBlau^{®} ist.

4. Verfahren nach einem der Ansprüche 1-3, wobei die Zellen mit dem Testagenz an Tagen 0, 2, 4 und 6 in Kontakt gebracht werden.

5. Verfahren nach einem der Ansprüche 1-4, wobei die Zellen in einer Dichte ausplattiert werden, die ungefähr 40.000 Zellen pro Well in einer 96-Wellplatte entspricht.

6. Verfahren nach einem der Ansprüche 1-5, das mit Leukämie-Inhibitor Faktor (LIF) als Positivkontrolle durchgeführt wird.

7. Verfahren nach einem der Ansprüche 1-6, wobei der Bereich des zentralen Nervensystems der Hippocampus oder die subventrikuläre Zone ist.

8. Assay zum Identifizieren eines neurogenen Agenz, wobei der Assay umfasst:
eine stabile humane neurale Vorläufer-Zelllinie, wobei
(i) die Zelllinie aus einem Bereich des zentralen Nervensystems abgeleitet ist,
(ii) die Zelllinie mit wenigstens zehn Verdoppelungen entwickelt wurde, und
(iii) die Zelllinie sowohl in Neuronen als auch Gliazellen bei einer Differenzierung ohne Mitogen differenziert;
eine Assayplatte, die zuvor mit extrazellulären Matrixproteinen beschichtet wurde, worin undifferenzierte neurale Vorläuferzellen der Zelllinie in einem Medium ohne Serum und ohne Mitogen kultiviert werden;
ein Testagenz; und
Antikörper, die mit Neuronen immunreaktiv sind, um die Neuronenanzahl festzustellen, und ein Fluoreszenzfarbstoff zum Erfassen der Proliferation, wobei das Testagenz
ein fusioniertes Imidazol wie in Strukturformel 1 beschrieben ist, oder
ein Aminopyrimidin wie in Strukturformel 2 beschrieben ist, oder
ein Nikotinamid wie in Strukturformel 3 beschrieben ist, oder
ein Aminomethylphenoxypiperidin wie in Strukturformel 4 beschrieben ist, oder
ein Aryloxypiperidin wie in Strukturformel 5 beschrieben ist.

9. Assay nach Anspruch 8, wobei die Antikörper gegen Neuronen immunreaktiv mit Typ III β-Tubulin, MAP2c oder TUJ1 sind.

10. Assay nach Anspruch 8 oder 9, wobei der Fluoreszenzfarbstoff AlamarBlau^{®} ist.

11. Assay nach einem der Ansprüche 8-10, wobei die undifferenzierten neuralen Vorläuferzellen in einer Dichte ungefähr 40.000 Zellen pro Well in einer 96-Wellplatte ausplattiert werden.

12. Assay nach einem der Ansprüche 8-11, der weiterhin LIF als Positivkontrolle beinhaltet.

13. Assay nach einem der Ansprüche 9-12, wobei der Bereich des zentralen Nervensystems der Hippocampus oder die subventrikuläre Zone ist.

## Revendications

1. Procédé d'identification d'un agent neurogène, comprenant :
la fourniture d'une lignée de cellules progénitrices neuronales humaines stables, dans lequel
(i) la lignée cellulaire est dérivée d'une zone du système nerveux central,
(ii) la lignée cellulaire a été multipliée pendant au moins dix doublements cellulaires, et
(iii) la lignée cellulaire génère à la fois des neurones et des cellules gliales lors de la différenciation en l'absence d'agent mitogène ;
l'étalement des cellules de ladite lignée cellulaire dans un récipient de culture avec un milieu sans sérum, sans agent mitogène ;
la mise en contact des cellules avec un agent à tester ;
l'incubation des cellules en présence de l'agent à tester pendant au moins sept jours ; et
après lesdits 7 jours, l'évaluation du nombre de neurones par une immunocoloration avec des anticorps contre les neurones et du nombre de cellules qui respirent en utilisant un colorant fluorescent en tant que mesure de la prolifération, dans lequel une augmentation d'au moins 30 % de la prolifération par rapport au témoin véhicule et une augmentation d'au moins 10 % des nombres de neurones par rapport au témoin véhicule identifie le composé à tester comme un agent neurogène, dans lequel l'agent à tester
est un imidazole condensé, tel que décrit dans la formule développée 1, ou
est une aminopyrimidine, tel que décrit dans la formule développée 2 ; ou
est un nicotinamide, tel que décrit dans la formule développée 3 ; ou
est une aminométhyle phénoxypipéridine, tel que décrit dans la formule développée 4 ; ou
est une aryloxypipéridine, tel que décrit dans la formule développée 5.

2. Procédé selon la revendication 1, dans lequel les anticorps contre les neurones sont immunoréactifs avec la ?-tubuline de type III, la MAP2c ou le TUJ1.

3. Procédé selon la revendication 1 ou 2, dans lequel le colorant fluorescent est l'AlamarBlue®.

4. Procédé selon l'une quelconque des revendications 1 à 3, dans lequel les cellules sont mises en contact avec l'agent à tester aux jours 0, 2, 4 et 6.

5. Procédé selon l'une quelconque des revendications 1 à 4, dans lequel lesdites cellules sont étalées à une densité correspondant à environ 40 000 cellules par puits d'une plaque de 96 puits.

6. Procédé selon l'une quelconque des revendications 1 à 5, qui est réalisé en utilisant le facteur inhibiteur de leucémie (LIF) en tant que témoin positif.

7. Procédé selon l'une quelconque des revendications 1 à 6, dans lequel la zone du système nerveux central est l'hippocampe ou une zone sous-ventriculaire.

8. Dosage destiné à identifier un agent neurogène, ledit dosage comprenant :
une lignée de cellules progénitrices neuronales humaines stables, dans laquelle
(i) la lignée cellulaire est dérivée d'une zone du système nerveux central,
(ii) la lignée cellulaire a été multipliée pendant au moins dix doublements cellulaires, et
(iii) la lignée cellulaire se différencie à la fois en neurones et en cellules gliales lors de la différenciation en l'absence d'agent mitogène ;
une plaque de dosage pré-enduite avec des protéines de la matrice extracellulaire, dans laquelle les cellules progénitrices neuronales non différenciées de ladite lignée cellulaire sont cultivées dans un milieu sans sérum, sans agent mitogène ;
un agent à tester ; et
des anticorps immunoréactifs avec les neurones pour l'évaluation du nombre de neurones et
un colorant fluorescent pour évaluer la prolifération, dans lequel l'agent à tester est un imidazole condensé, tel que décrit dans la formule développée 1, ou
est une aminopyrimidine, tel que décrit dans la formule développée 2 ; ou
est un nicotinamide, tel que décrit dans la formule développée 3 ; ou
est une aminométhyle phénoxypipéridine, tel que décrit dans la formule développée 4 ; ou
est une aryloxypipéridine, tel que décrit dans la formule développée 5.

9. Dosage selon la revendication 8, dans lequel les anticorps contre les neurones sont immunoréactifs avec la ?-tubuline de type III, la MAP2c ou le TUJ1.

10. Dosage selon la revendication 8 ou 9, dans lequel le colorant fluorescent est l'AlamarBluc®.

11. Dosage selon l'une quelconque des revendications 8 à 10, dans lequel les cellules précurseurs neuronales non différenciées sont étalées à une densité d'environ 40 000 cellules par puits dans une plaque de 96 puits.

12. Dosage selon l'une quelconque des revendications 8 à 11, qui comprend en outre du LIF comme témoin positif.

13. Dosage selon les revendications 9 à 12, dans lequel la zone du système nerveux central est l'hippocampe ou une zone sous-ventriculaire.
